# EUROPEAN PATENT SPECIFICATION

(11) **EP 4 125 938 B1**
(45) Date of publication and mention of the grant of the patent: **11.09.2024**
(21) Application number: 21722586.1
(22) Date of filing: 24.03.2021
(51) Int. Cl.: A61K 31/795, A61P 31/14

(54) **USE OF SULFONATED POLYSTYRENE DERIVATIVES IN THE TREATMENT AND/OR PROPHYLAXIS OF INFECTION CAUSED BY ZIKA VIRUS**
VERWENDUNG VON SULFONIERTEN POLYSTYROLDERIVATEN ZUR BEHANDLUNG UND/ODER PROPHYLAXE VON DURCH ZIKA-VIRUS VERURSACHTEN INFEKTIONEN
UTILISATION DE DÉRIVÉS DE POLYSTYRÈNE SULFONÉ DANS LE TRAITEMENT ET/OU LA PROPHYLAXIE D'UNE INFECTION PROVOQUÉE PAR LE VIRUS ZIKA

(30) Priority: 03.04.2020 PL 43344320
(43) Date of publication of application: 08.02.2023
(73) Proprietor: Uniwersytet Jagiellonski, 31-007 Krakow (PL)
(72) Inventor: PYRC, Krzysztof, 31-231 Krakow (PL); OBLOZA, Magdalena, 31-572 Krakow (PL); NOWAKOWSKA, Maria, 30-433 Krakow (PL); BOTWINA, Pawel, 37-150 Krakow (PL); SZCZUBIALKA, Krzysztof, 32-442 Krzywaczka (PL)
(74) Representative: Witek, Rafal
(86) International application number: PCT/PL2021/000016
(87) International publication number: WO 2021/201705

(56) References cited:
- WO-A1-2017/190193
- TAN CHEE WAH ET AL: "Polysulfonate suramin inhibits Zika virus infection", ANTIVIRAL RESEARCH, ELSEVIER BV, NL, vol. 143, 27 April 2017 (2017-04-27), pages 186 - 194, XP085032225, ISSN: 0166-3542, DOI: 10.1016/J.ANTIVIRAL.2017.04.017
- HEROLD BETSY C. ET AL: "Poly(Sodium 4-Styrene Sulfonate): An Effective Candidate Topical Antimicrobial for the Prevention of Sexually Transmitted Diseases", JOURNAL OF INFECTIOUS DISEASES, vol. 181, no. 2, 1 February 2000 (2000-02-01), US, pages 770 - 773, XP055823240, ISSN: 0022-1899, Retrieved from the Internet <URL:https://watermark.silverchair.com/181-2-770.pdf?token=AQECAHi208BE49Ooan9kkhW_Ercy7Dm3ZL_9Cf3qfKAc485ysgAAAtEwggLNBgkqhkiG9w0BBwagggK-MIICugIBADCCArMGCSqGSIb3DQEHATAeBglghkgBZQMEAS4wEQQMAxly943NiHtqlSSeAgEQgIIChEjwRU_Jjng5Oh5NYXdoGNCijrXsVTAWc-HPvYI2Q6kWYtWhlYhddWwkSU8JaFDfbE1mx7V8lvV4FEg_jYnOSruC0T> DOI: 10.1086/315228
- SYNOWIEC ALEKSANDRA ET AL: "Cat flu: Broad spectrum polymeric antivirals", ANTIVIRAL RESEARCH, ELSEVIER BV, NL, vol. 170, 17 July 2019 (2019-07-17), XP085839617, ISSN: 0166-3542, [retrieved on 20190717], DOI: 10.1016/J.ANTIVIRAL.2019.104563
- BOTWINA PAWEL ET AL: "In Vitro Inhibition of Zika Virus Replication with Poly(Sodium 4-Styrenesulfonate)", VIRUSES, vol. 12, no. 9, 23 August 2020 (2020-08-23), pages 926, XP055823235, DOI: 10.3390/v12090926
- MURUGAN KADARKARAI ET AL: "Poly(Styrene Sulfonate)/Poly(Allylamine Hydrochloride) Encapsulation of TiO2Nanoparticles Boosts Their Toxic and Repellent Activity Against Zika Virus Mosquito Vectors", JOURNAL OF CLUSTER SCIENCE, SPRINGER NEW YORK LLC, US, vol. 29, no. 1, 27 September 2017 (2017-09-27), pages 27 - 39, XP036398821, ISSN: 1040-7278, [retrieved on 20170927], DOI: 10.1007/S10876-017-1300-3

## Description

The invention relates to a sulfonated polystyrene derivative (PSS), in particular poly(sodium 4-styrene sulfonate) (PSSNa), for use in the treatment and/or prophylaxis of infection caused by Zika virus (ZIKV), as well as the use of such sulfonated derivative as a medicament or an active substance in the treatment/prophylaxis of ZIKV infections.

ZIKV, belonging to the *Flaviviridae* family, was first isolated in 1947 from a sample obtained from macaque¹. For many years, however, it did not attract special attention of scientists due to very rare and often asymptomatic infections in humans. However, the picture began to change from 2007, when the ZIKV epidemic broke out in Micronesia (Yap Island)². Another epidemic broke out six years later in the Polynesian region, where the disease affected nearly 20,000 people³. The next wave of cases took place in Brazil and affected even 1,300,000 people. ZIKV has spread around the world in a relatively short time, and the World Health Organization (WHO) has classified it as a pathogen posing an international threat to public health. 2019 WHO data indicates that Zika virus transmission has been recorded in 87 countries and territories. With increasing numbers of cases it has been observed that although the disease itself is relatively mild, its consequences can be very serious. This applies in particular to the infection of women during pregnancy as it may lead to fetal microcephaly⁴. ZIKV is an arbovirus that requires a vector to spread - the *Aedes* mosquito⁴, however, infection can also occur through direct contact with an infected person, e.g. during sexual contacts. Many birth defects and disabilities, known as congenital Zika syndrome, are found in children of women who are infected during pregnancy.^{6,7}. The most common defects include microcephaly, damage to the brain tissue, damage to the back of the eye, macular scarring, congenital contractures such as clubfoot or arthrogryposis, and hypertonia that restrict body movement shortly after birth⁸. In adults, ZIKV infection is also associated with complications of the nervous system, such as Guillain-Barre syndrome⁴.

Although ZIKV poses a serious health threat, there is no effective vaccine and no ZIKV-specific antiviral treatment is available. In case of infection, only supportive treatment is recommended. The polysulfonate suramin was found to inhibit Zika virus infection¹¹.

Therefore, there is a need for a new inhibitory drug which contains as an active ingredient a compound that effectively inhibits ZIKV replication, has low toxicity, and its use would reduce the risk of complications and alleviate the symptoms of infection.

The first object of the invention is poly(sodium 4-styrene sulfonate) for use in the treatment and/or prophylaxis of infection caused by Zika virus.

In a preferred embodiment of the invention, the molecular weight of poly(sodium 4-styrene sulfonate) is between 1.1 kDa and 3160 kDa.

In another preferred embodiment of the invention, the molecular weight of poly(sodium 4-styrene sulfonate) is 3160 kDa.

In a further embodiment of the invention, the concentration of poly(sodium 4-styrene sulfonate) is from 0.5 mg/mL to 2 mg/mL.

In yet another embodiment of the invention, the concentration of poly(sodium styrene-4-sulfonate) is 1 mg/mL.

The aim of the research was to develop a new inhibitor that will alleviate the symptoms of ZIKV infection and reduce the risk of complications. The most effective polymers were PSSNa polymers (poly(sodium 4-styrene sulfonate)), which practically completely inhibited the replication of the virus *in vitro.* At the same time, the polymers used were characterized by low toxicity to human glioblastoma (U251) cells and monkey kidney (Vero PZH) cells susceptible to Zika virus infection. The conducted research has shown that high molecular weight polymers are the most effective. Mechanistic tests have shown that PSSNa binds ZIKV and thus blocks their ability to infect host cells.

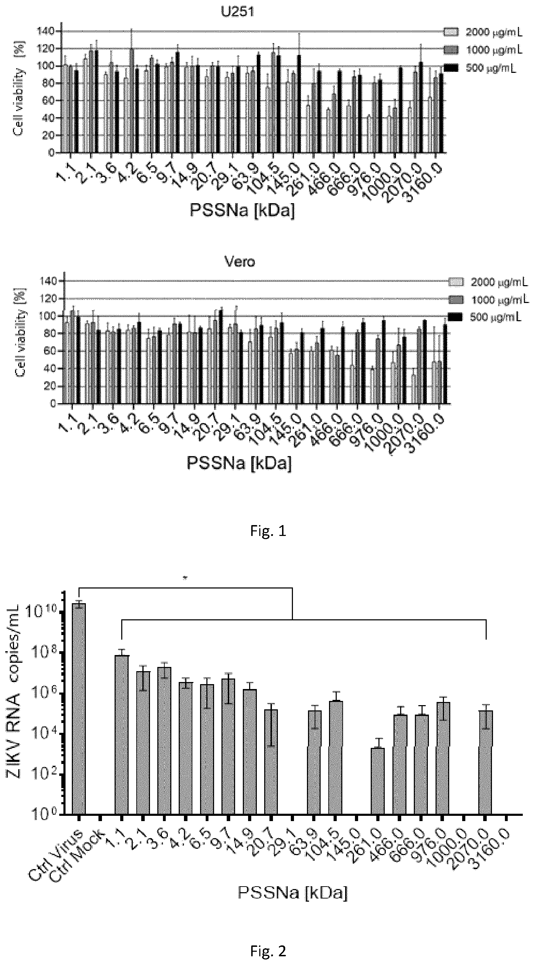

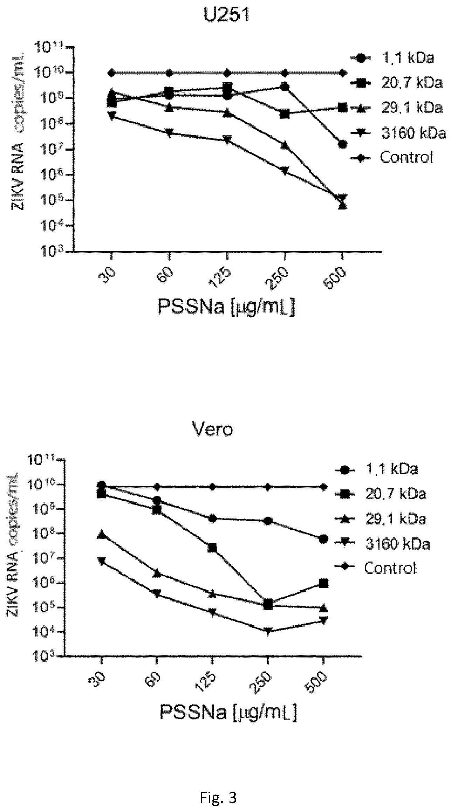

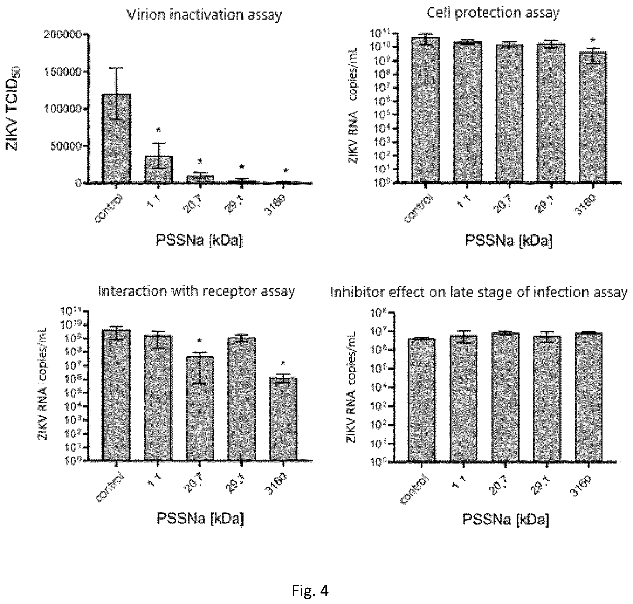

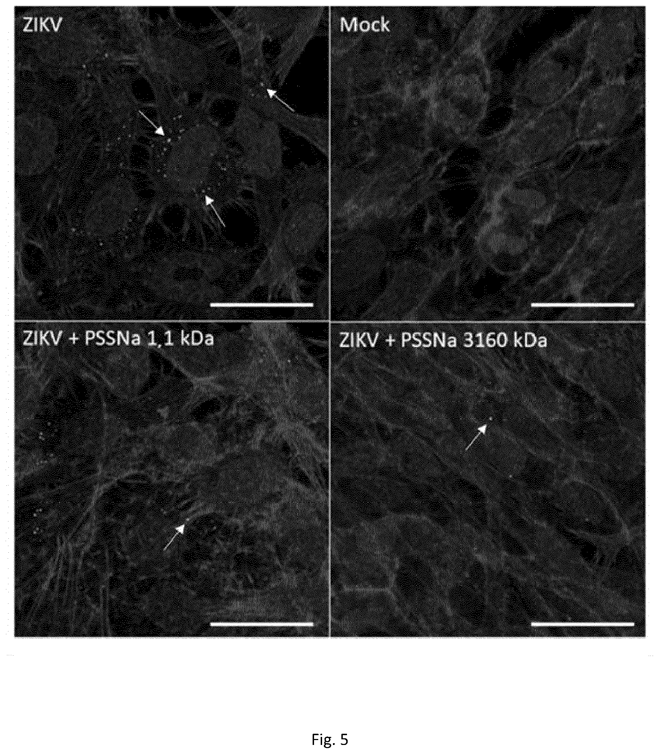

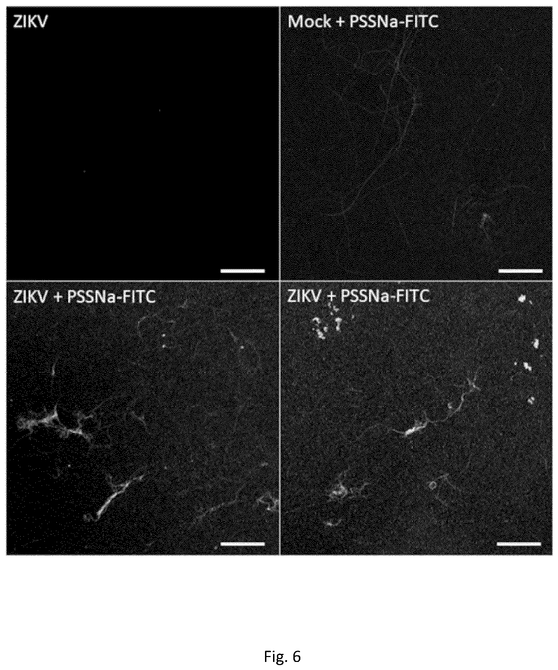

Embodiments of the invention are illustrated in Figures 1-6. Figure 1 shows the cytotoxicity of PSSNa of different molecular weights at concentrations of 2000, 1000 and 500 µg/mL, determined by the XTT assay on U251 and Vero PZH cells. All experiments were performed in triplicate. Figure 2 shows mean values with standard deviations (error bars) representing the inhibition of the ZIKV replication cycle by PSSNa polymers of different molecular weights in U251 cells. Infection inhibition was assessed using quantitative real time PCR (RT-qPCR). The polymers were present in the medium throughout the ZIKV replication cycle. Data are shown as logarithmic values of ZIKV RNA copy number per milliliter. All experiments were performed in triplicate. The results are presented as mean values with standard deviations (error bars). An asterisk indicates values statistically different from the control (P < 0.05). Figure 3 shows the inhibition of the ZIKV replication cycle by PSSNa in different cell types. Infection inhibition was assessed using RT-qPCR. U251 and Vero PZH cells were infected in the presence of the appropriate concentration of PSSNa for 4 and 3 days, respectively Data are shown as logarithmic values of ZIKV RNA copy number per milliliter. The results are presented as mean values of at least three replicates. Figure 4 shows the antiviral activity of PSSNa of different molecular weights at different stages of the ZIKV infection cycle. The names of the relevant tests are marked above the graphs. Infection inhibition was assessed using RT-qPCR. All experiments were performed in triplicate. The results are presented as mean values with standard deviations (error bars). Asterisks indicate values statistically different from the control (P < 0.05). Figure 5 shows PSSNa blocking ZIKV from attaching to host cells. PZH Vero cells were infected with ZIKV (TCID₅₀ = 10000/mL) in the presence of 250 µg/mL PSSNa. Cells were fixed 2 hours after incubation at 4 °C and confocal images were collected. Scale bar = 100 µm. Fig. 6 shows the interaction of PSSNa with ZIKV molecules. PSSNa-FITC or PBS were applied to collagen coated coverslips. ZIKV was then added and incubated for 1 hour. Slides were fixed, immunostained with antibodies specifically detecting the ZIKV E protein and imaged. Scale bar = 100 µm.

PSSNa is a cheap and easy to synthesize polymer. It can be obtained by radical polymerization with a very wide range of molecular weights. It is also a polymer that has already been used clinically. As a drug in the form of an oral or rectal suspension, it is used in the treatment of hyperkalemia, i.e. excessively high levels of potassium in the plasma (e.g. Kayexelate, Kionex, Kalexelate preparations). It is not absorbed from the digestive system.

### Example 1

Effect of poly(sodium 4-styrene sulfonate) (PSSNa) of different molecular weights on cell viability.

Commercially available (Pressure Chemicals and Sigma-Aldrich) poly(sodium 4-styrene sulfonates) of various molecular weights and low dispersion coefficient (used as analytical standards in gel permeation chromatography (GPC)) were used in the research.

### Methods

The cytotoxicity of the polymers was determined using the XTT Viability Assay Kit (Biological Industries, Israel). The test allows a quantitative determination of the activity of cell metabolism. Only living, metabolically active cells are able to transform the tetrazolium substrate (2,3-bis-(2-methoxy-4-nitro-5-sulfenyl)-(2H)-tetrazolium-5-carboxanilide, XTT) into its colored derivativethe concentration of which, measured by absorbance at 480 nm, correlates with the number of viable cells and their viability. Permissive cell lines, i.e. human glioblastoma cells (U251) and monkey kidney cells (Vero PZH), were used to carry out the experiment. Cells were grown for 48 h in DMEM (Dulbecco's Modified Eagle's Medium) supplemented with 3% FBS (heat-inactivated fetal bovine serum), penicillin, streptomycin and gentamicin. Aqueous PSSNa solutions of different molecular weights or, in the case of control samples, water were added to the medium. After the cells were incubated in a 96-well plate for 4 days, the medium was removed and 100 µL of fresh medium and 20 µL of activated XTT were added to each well of the plate. After 2 h of incubation, the supernatant was transferred to a transparent 96-well plate and the absorbance at 480 nm was measured using a spectrophotometer in a standard way. The values obtained were normalized to the absorbance measured for control cells (without PSSNa), which were assigned 100% viability. Aqueous solutions of 19 PSSNa polymers of different molecular weights (1.1; 2.1; 3.6; 4.2; 6.5; 9.7; 14.9; 20.7; 29.1; 63.9; 104.5; 145; 261; 466; 666; 976; 1000; 2070 and 3160 kDa) were tested at 3 different concentrations (0.5 mg/mL, 1 mg/mL and 2 mg/mL). The results of the experiment are shown in Fig. 1.

### Results

The obtained results indicate low cytotoxicity of the polymers at the concentration of 0.5 mg/mL and 1 mg/mL (Fig. 1) for both tested cell lines.

### Example 2

Effect of poly(sodium 4-styrene sulfonate) (PSSNa) on ZIKV infection.

### Methods

In order to determine the antiviral activity of poly(sodium 4-styrene sulfonate) (PSSNa) against ZIKV (strain H/PF/2013), a test for inhibition of ZIKV infection by PSSNa was performed. In this experiment, the polymer was present in the medium at every stage of viral infection. Completely confluent U251 cells were used in the experiment 48 h after plating in a 96-well plate. The medium was removed and 100 µL of fresh medium was added with the addition of 3% FBS (heat-inactivated fetal bovine serum), penicillin, streptomycin, gentamicin and PSSNa polymers of various molecular weights (1 mg/mL for PSSNa of 19 different molecular weights and 0.5 mg/mL for PSSNa selected for testing the mechanism of action described in Example 3). Control samples did not contain PSSNa.

ZIKV was added to the cells in an amount allowing to reach a final TCID₅₀ (50% tissue culture infective dose) titer = 400/mL. Plates were incubated for 2 h at 37 °C, then cells were washed twice with PBS solution to remove unbound viral particles. Finally, 100 µL of a polymer solution in a culture medium was added to each well, and the cells were incubated for 4 days. After this time, the supernatant was harvested for viral RNA copy number evaluation using quantitative real time PCR (RT-qPCR).

Viral RNA isolation was performed using a commercially available RNA isolation kit (Viral DNA/RNA Isolation Kit, A&A Biotechnology, Poland) according to the protocol provided by the manufacturer. The RNA isolated in this way was subjected to reverse transcription (RT) and quantitative real-time PCR (qPCR).

Briefly, the RT-qPCR reaction was performed as follows. 3 µL of the isolated viral RNA was reverse transcribed into the cDNA template and amplified in a 10 µL reaction containing 1 × GoScript^{™} RT Mix for 1-Step RT-qPCR, 1 × GoTaq Probe qPCR Master Mix with dUTP, 100 nM specific probe labeled with 6-carboxyfluorescein (FAM) and 6-carboxytetramethylrhodamine (TAMRA) (5'-FAM-CGGCATACAGCATCAGGTGCATAGGAG-TAMRA-3'), and 450 nM of each primer (5'-TTGGTCATGATACTGCTGATTGC-3' and 5'-CCTTCCACAAAGTCCCTATTGC-3'). The reaction was carried out in a thermal cycler (CFX96 Touch^{™} Real-197 Time PCR Detection System, Bio-Rad) under the following conditions: 45 °C for 15 min (reverse transcription), 95 °C for 2 min, then 40 cycles of 15 seconds at 95 °C and 30 seconds at 60 °C.

Appropriate standards were prepared to assess the initial number of viral RNA molecules in the sample. The cDNA transcribed sequence fragment was amplified using the primers described above. The DNA thus obtained was cloned into the pTZ57R/T plasmid (Thermo Scientific, Poland) using the InsTAclone PCR Cloning Kit (Thermo Scientific, Poland). Transformation of the *E. coli* TOP10 strain (Life Technologies, Poland) and amplification of the plasmid vector were performed in a standard manner. The plasmid was then purified using the GeneJET Plasmid Miniprep Kit (Thermo Scientific, Poland) and linearized by digestion with the *Kpnl* restriction enzyme. The concentration of linearized DNA was assessed by spectrophotometric measurement and the copy number per milliliter was calculated. Six 10-fold serial dilutions were used as the template in the real-time PCR reaction. The ability of the polymers to inhibit ZIKV proliferation was determined as the decrease in the log of the number of viral RNA copies per milliliter of medium.

### Results

In the first experiment (Figure 2), U251 cells were infected with ZIKV in the presence of each of the tested PSSNa polymers of 19 different molecular weights. RT-qPCR analyses showed very strong inhibition of ZIKV replication at a concentration of 500 µg/mL. The inhibitory effect has also been found to be related to the molecular weight of the polymer. High molecular weight PSSNa showed more inhibitory activity (more than 4 logs inhibition), while lower molecular weight polymers showed less inhibition (2-4 logs decrease). With some polymers the inhibition was so strong that the viral RNA copy number was below the lower detection limit of the RT-qPCR method (approximately 1000 copies/mL).

Following these promising preliminary experiments, further experiments were performed using 4 polymers covering a wide range of molecular weights (1.1, 20.7, 29.1 and 3,160 kDa). A viral replication inhibition test was performed on U251 and Vero PZH cells to see if the effect was cell type dependent. The experiments showed a very similar pattern of inhibition in U251 and Vero PZH cells, suggesting that PSSNa acts directly on the components of ZIKV and not on cellular metabolism (Fig. 3). Again, a correlation was observed between the molecular weight of PSSNa and the inhibition efficiency of ZIKV, i.e. the higher molecular weight PSSNa polymers (29.1 and 3160 kDa) inhibited ZIKV more effectively than the lower molecular weight polymers. For U251 cells, strong dose-dependent viral inhibition was observed only for PSSNa with molecular weights of 29.1 and 3160 kDa, at the IC₅₀ dose of 14.3 and 8.8 µg/mL, respectively. In contrast, in Vero PZH cells, all tested PSSNa polymers inhibited ZIKV infection in a dose-dependent manner, and their effectiveness increased with increasing their molecular weight. The viral replication inhibition test also showed complete inhibition of replication of the ZIKV MR-776 strain in primary human skin fibroblasts (HSF) for all PSSNa polymers tested (1.1, 20.7, 29.1 and 3160 kDa) at 250 µg/mL ( data not shown).

The drug PSSNa for use in the treatment or prophylaxis of Zika virus infection may be in the form of an intravenous solution.

### Example 3

Fluorescein-labeled PSSNa was obtained according to the procedure previously described in the literature.¹⁰

Determination of the mechanism of the antiviral action of PSSNa polymers.

### Methods

In order to determine the mechanism of action of PSSNa and to identify the stage in the replication cycle at which ZIKV infection is suppressed, the functional tests described below were performed at a polymer concentration of 0.5 mg/mL. Four different PSSNa polymers of different molecular weights (1.1 kDa, 20.7 kDa, 29.1 kDa and 3160 kDa) were used in the research. The following cellular tests were performed:

### 1. An assay for virion inactivation by PSSNa

Concentrated ZIKV was incubated in the presence of PSSNa at a concentration of 0.5 mg/mL. After incubation, samples were diluted 1000 × to eliminate the effect of PSSNa on the cells themselves. The prepared preparations were analyzed using titration according to Reed and Muench methods⁹ by preparing 10 serial fivefold dilutions of virus and infecting the U251 cell monolayer in 8 replicates. After this time, the appearance of the cytopathic effect due to ZIKV infection was assessed using an optical microscope. The number of wells where CPE occurred correlates linearly with the log TCID₅₀ value.

### 2. An assay for cell protection by PSSNa

The effect of PSSNa on cells was assessed by incubating U251 cells for 2 h with PSSNa (0.5 mg/mL) at 37 °C. After this time, the polymer was washed and the cells were infected with ZIKV (TCID₅₀ = 400/mL). After 5 days of incubation (37 °C; 5% CO₂), ZIKV replication analysis was performed using RT qPCR. The effect of PSSNa on cells was assessed on the basis of the changes in viral replication.

### 3. An assay for PSSNa interaction with receptor

The effect of PSSNa as an inhibitor of virus binding to the cell surface receptor was assessed by adsorbing the virus to U251 cells in the presence of the inhibitor and then removing it from the medium. The virus mixture (TCID₅₀ = 400/mL, 4 °C), the control sample with PSSNa (0.5 mg/mL, 4 °C) or the control sample (without PSSNa) were added to the cells cooled to 4 °C. The samples were incubated for 1 h at 4 °C to allow the virus to bind to the receptor and at the same time to block internalisation into the cell (cellular transport was blocked by low temperature). Samples were washed three times with PBS, then culture medium was added and incubated for 5 days (37 °C; 5% CO₂). After this time, ZIKV replication analysis was performed using RT qPCR. Based on the changes in viral replication, the effect of PSSNa on the binding of the virus to the cell receptor was assessed.

### 4. An assay for PSSNa influence on late stages of infection

The effect of PSSNa as an inhibitor of the late stages of infection was assessed by infecting U251 cells without PSSNa followed by incubation in the presence of PSSNa after washing out the virus. Thus, at the time of viral adsorption to cells and its internalisation, PSSNa was not present. Cells were incubated in medium containing ZIKV (TCID₅₀ = 400/mL) or a control sample for 2 h at 37 °C. After this time, the cells were washed three times with PBS, and PSSNa supplemented culture medium (0.5 mg/mL) or control was applied and incubated for 12 hours, which is the time of one ZIKV replication cycle (37 °C; 5% CO₂). After this time, ZIKV replication analysis was performed using RT qPCR. Based on the changes in viral replication, the effect of PSSNa on the late stages of infection was assessed.

### Confocal microscopy

Cells were seeded on coverslips in 12-well plates (TPP, Switzerland). Following the experiment, cells were fixed with 4% formaldehyde, permeabilized with 0.1 Triton X-100 and blocked by incubation with 5% bovine serum albumin (BSA, Bioshop, Canada) in PBS for 2 hours. The slide preparations were then incubated for 2 hours with 1 µg/mL rabbit anti-ZIKV envelope antibody (GeneTex, USA). After washing 3 times with PBS, cells were incubated for 1 hour with 2.5 µg/mL goat anti-rabbit secondary antibody conjugated to Atto 488 fluorophore and/or Alexa Fluor 647 conjugated phalloidin (0.2 U/mL, Invitrogen, Poland) staining cell actin. Specimens were then washed three times with PBS and the cell nuclei (DNA) was stained with DAPI (0.1 µg/mL, Sigma-Aldrich, Poland) for 20 minutes at room temperature. Coverslips were washed several times with PBS and mounted on slides using ProLong Diamond Antifade Mountant medium (Life Technologies, Poland). Images were obtained with a Zeiss LSM 710 confocal microscope (Carl Zeiss Microscopy GmbH; version 8.1) using the ZEN 2012 SP1 software (Carl Zeiss Microscopy GmbH; black edition, version 8.1.0.484).

### Results

The results of the functional tests suggest that PSSNa works by directly interacting with the Zika virus. This is evidenced by the very strong inhibition of the infectivity of the sample after incubation with the PSSNa solution, presented in Fig. 4 - the results of the virion inactivation test. An increase in the effectiveness of the antiviral effect was also observed here with increasing the molecular weight of the polymer. The 3160 kDa PSSNa reduced the TCID₅₀/mL value by almost 3 logs, while the 1.1 kDa PSSNa reduced the value by only 0.5 logs. However, significant inhibition was observed for the 2 PSSNa polymers (with molecular weights of 20.7 and 3160 kDa) also in the virus attachment assay (test III). Therefore, an additional independent mechanism of action by which PSSNa blocks the interaction between the ZIKV particle and the cellular receptor cannot be ruled out. However, this effect is not due to the direct interaction of PSSNa with the receptor since no decrease in ZIKV replication was observed in the cell protection assay (test II) in which polymers were incubated with cells prior to infection. No inhibition of ZIKV numbers was observed in PSSNa-treated cells after the initial stages of viral replication. Therefore, it can be assumed that the antiviral activity of PSSNa is mainly due to its direct interaction with ZIKV particles, which prevents the virus from attaching to the host cells.

Confocal images confirmed that PSSNa prevents the virus from attaching to the cell. The cells were incubated with the virus and PSSNa at 4 °C for 2 h. At low temperature, ZIKV could attach to the cell receptor, but could not be internalized into the cell. The obtained images showed that both PSSNa tested (i.e. with molecular weights of 1.1 and 3160 kDa) significantly reduced the number of virions (indicated by white arrows) bound to the cell (Fig. 5). To directly observe the interaction between ZIKV and PSSNa, a ZIKV binding assay was performed using fluorescein-labeled PSSNa (PSSNa-FITC) immobilized on collagencoated coverslips. A significant increase in the ZIKV envelope signal was observed compared to the control without PSSNa-FITC. The confocal images thus show a strong interaction between PSSNa-FITC and ZIKV (Fig 6).

### Literature

(1) Dick, G. W. A. Zika Virus (I). Isolations and Serological Specificity. 1952, 46 (5), 509-520.
(2) Lanciotti, R. S.; Kosoy, O. L.; Laven, J. J.; Velez, J. O.; Lambert, A. J.; Johnson, A. J.; Stanfield, S. M.; Duffy, M. R. Genetic and Serologic Properties of Zika Virus Associated with an Epidemic, Yap State, Micronesia, 2007. 2008, 14 (8), 1232-1239.
(3) Musso, D.; Bossin, H.; Mallet, H. P.; Besnard, M.; Broult, J.; Baudouin, L.; Levi, J. E.; Sabino, E. C.; Ghawche, F.; Lanteri, M. C.; Baud, D. Zika Virus in French Polynesia 2013-14: Anatomy of a Completed Outbreak. The Lancet Infectious Diseases. 2018.
(4) Kraemer, M. U. G.; Sinka, M. E.; Duda, K. A.; Mylne, A.; Shearer, F. M.; Brady, O. J.; Messina, J. P.; Barker, C. M.; Moore, C. G.; Carvalho, R. G.; Coelho, G. E.; Van Bortel, W.; Hendrickx, G.; Schaffner, F.; Wint, G. R. W.; Elyazar, I. R. F.; Teng, H.-J.; Hay, S. I. The Global Compendium of Aedes Aegypti and Ae. Albopictus Occurrence. Sci. data 2015, 2, 150035.
(5) Musso, D.; Roche, C.; Robin, E.; Nhan, T.; Teissier, A.; Cao-Lormeau, V. M. Potential Sexual Transmission of Zika Virus. Emerg. Infect. Dis. 2015, 21 (2), 359-361.
(6) Baud, D.; Gubler, D. J.; Schaub, B.; Lanteri, M. C.; Musso, D. An Update on Zika Virus Infection. The Lancet. 2017.
(7) Wheeler, A. C. Development of Infants with Congenital Zika Syndrome: What Do We Know and What Can We Expect? Pediatrics 2018**.**
(8) Rice, M. E.; Galang, R. R.; Roth, N. M.; Ellington, S. R.; Moore, C. A.; Valencia-Prado, M.; Ellis, E. M.; Tufa, A. J.; Taulung, L. A.; Alfred, J. M.; Pérez-Padilla, J.; Delgado-López, C. A.; Zaki, S. R.; Reagan-Steiner, S.; Bhatnagar, J.; Nahabedian, J. F.; Reynolds, M. R.; Yeargin-Allsopp, M.; Viens, L. J.; Olson, S. M.; Jones, A. M.; Baez-Santiago, M. A.; Oppong-Twene, P.; VanMaldeghem, K.; Simon, E. L.; Moore, J. T.; Polen, K. D.; Hillman, B.; Ropeti, R.; Nieves-Ferrer, L.; Marcano-Huertas, M.; Masao, C. A.; Anzures, E. J.; Hansen, R. L.; Pérez-Gonzalez, S. I.; Espinet-Crespo, C. P.; Luciano-Román, M.; Shapiro-Mendoza, C. K.; Gilboa, S. M.; Honein, M. A. Vital Signs: Zika-Associated Birth Defects and Neurodevelopmental Abnormalities Possibly Associated with Congenital Zika Virus Infection - U.S. Territories and Freely Associated States, 2018. Morb. Mortal. Wkly. Rep. 2018**.**
(9) Reed, L. J.; Muench, H. A Simple Method of Estimating Fifty per Cent Endpoints. Am. J. Epidemiol. 1938, 27 (3), 493-497.
(10) Sohn, D., Russo, P.S., Davila, A., Poche, D.S., McLaughlin, M. L., J. Colloid Interface Sci. 1996, 17(1), 31-44.
(11) Chee Wah Tan , I-Ching Sam , Wei Lim Chong , Vannajan Sanghiran Lee , Yoke Fun Chan, Polysulfonate suramin inhibits Zika virus infection, Antiviral Res . 2017 Jul:143:186-194.

## Claims

1. Poly(sodium 4-styrene sulfonate) for use in the treatment and/or prophylaxis of infection caused by Zika virus.

2. The sodium salt for use according to claim 1, **characterized in that** the molecular weight of the poly(sodium 4-styrene sulfonate) is from 1.1 kDa to 3160 kDa.

3. The sodium salt for use according to claim 1, **characterized in that** the molecular weight of the poly(sodium 4-styrene sulfonate) is 3160 kDa.

4. The sodium salt for use according to claim 1 to 3, **characterized in that** the concentration of the poly(sodium 4-styrene sulfonate) is 0.5 mg/mL to 2 mg/mL.

5. The sodium salt for use according to claim 4, **characterized in that** the concentration of the poly(sodium 4-styrene sulfonate) is 1 mg/mL.

## Patentansprüche

1. Poly(natrium-4-styrolsulfonat) zur Verwendung bei der Behandlung und/oder Prophylaxe einer durch Zika-Virus verursachten Infektion.

2. Natriumsalz zur Verwendung gemäß Anspruch 1, **dadurch gekennzeichnet, dass** das Molekulargewicht des Poly(natrium-4-styrolsulfonats) 1,1 kDa bis 3160 kDa beträgt.

3. Natriumsalz zur Verwendung gemäß Anspruch 1, **dadurch gekennzeichnet, dass** das Molekulargewicht des Poly(natrium-4-styrolsulfonats) 3160 kDa beträgt.

4. Natriumsalz zur Verwendung gemäß Anspruch 1 bis 3, **dadurch gekennzeichnet, dass** die Konzentration des Poly(natrium-4-styrolsulfonats) 0,5 mg/ml bis 2 mg/ml beträgt.

5. Natriumsalz zur Verwendung gemäß Anspruch 4, **dadurch gekennzeichnet, dass** die Konzentration des Poly(natrium-4-styrolsulfonats) 1 mg/ml beträgt.

## Revendications

1. Poly(4-styrène sulfonate de sodium) à utiliser dans le traitement et/ou la prophylaxie d'une infection causée par le virus Zika.

2. Sel de sodium à utiliser selon la revendication 1, **caractérisé en ce que** le poids moléculaire du poly(4-styrène sulfonate de sodium) est de 1,1 kDa à 3160 kDa.

3. Sel de sodium à utiliser selon la revendication 1, **caractérisé en ce que** le poids moléculaire du poly(4-styrène sulfonate de sodium) est de 3160 kDa.

4. Sel de sodium à utiliser selon les revendications 1 à 3, **caractérisé en ce que** la concentration du poly(4-styrène sulfonate de sodium) est de 0,5 mg/mL à 2 mg/mL.

5. Sel de sodium à utiliser selon la revendication 4, **caractérisé en ce que** la concentration du poly(4-styrène sulfonate de sodium) est de 1 mg/mL.
